Europäisches Patentamt

European Patent Office

Office européen des brevets

(11) Publication number: **0 152 382**
**B1**

## EUROPEAN PATENT SPECIFICATION

(45) Date of publication of patent specification: **13.06.90**

(51) Int. Cl.⁵: **A 61 B 17/11**

(21) Application number: **85830036.1**

(22) Date of filing: **15.02.85**

(54) Circular mechanical anastomotic gun.

(30) Priority: **16.02.84 IT 1965384**

(43) Date of publication of application:
**21.08.85 Bulletin 85/34**

(45) Publication of the grant of the patent:
**13.06.90 Bulletin 90/24**

(84) Designated Contracting States:
**BE DE FR GB IT SE**

(56) References cited:
**WO-A-81/00668**
**WO-A-82/02824**
**FR-A-2 490 482**
**GB-A-2 016 991**

(73) Proprietor: **Pfizer Hospital Products Group, Inc.**
**235 East 42nd Street**
**New York New York 10017 (US)**

(72) Inventor: **Rosati, Riccardo**
**Via Livorno, 4**
**Milano (IT)**
Inventor: **Rebuffat, Carlo**
**Via Galilei, 17**
**Trento (IT)**

(74) Representative: **Mercurio, Franco et al**
**c/o Società Italiana Brevetti S.p.A. Via Carducci,**
**8**
**I-20123 Milano (IT)**

Courier Press, Leamington Spa, England.

# Description

The object of the present invention is a new mechanical gun suitable to perform circular anastomosis in hollow organs. Circular mechanical gun are known in the art, which utilize for joining the tissue edges of the resected hollow organs a system of metallic staples which are inserted from inside the hollow organ, into the overlapped tissues of the two hollow organ edges, and still rivetted inwards to achieve the anastomosis.

It is also known a circular mechanical anastomotic gun utilizing a particular system consisting of partially metallic and partially plastic flat rings kept together by a system of connecting nails with coaxial springs to achieve the anastomosis by a compression applied to the tissue edges of the specific dissected hollow organs.

This system is disclosed in the FR—A—2 490 482 where a circular cutter moves rigidly with said nails, so that the cutter advances cutting the rings and in the same time the nails remain in the organs to be anastomized.

The circular mechanical anastomotic gun of the present invention achieves the anastomosis in the digestive apparatus, using a compression device forming the object of a patent application EP—A—0 152 381 of the Applicants which has been filed at even date as the present application.

This anastomosis device comprises three coaxial elements of a particular shape which realize the compression of the hollow organ dissected tissues between the walls of two of such elements, said compression being applied suitably by inserting the third element into the second one.

After a certain period of time has elapsed, the compressed tissues necrotize and fall inside the hollow organ dragging together the device causing the compression: both are naturally eliminated whilst in that interval of time the anastomosis of the faced tissues immediately outside the area where the pressure is applied, occurs.

The mechanical gun for circular anastomosis, forming the object of the present invention is essentially formed by three parts:

1) A head portion consisting of two parts: the first one, at one end, having a broad size, the second one having a cylindrical shape.

The broad part carries a device able to perform the anastomosis by compression, and a cutting member which allows the mechanical gun to disengage from the organs when the suturing device assembly is realized, allowing also the withdrawal of the same from inside the hollow organ. The cylindrical part is formed by a central pivot which is the axis of the whole circular mechanical anastomotic gun and makes it possible to introduce more or less deeply the gun end portion inside the dissected hollow member.

The central pivot is surrounded by a system of concentric tubular elements which support: the outer, the intermediate element of the compression device; the median, the element of the compression device which inserts into the inter-mediate element and lastly the inner one, the cutting member respectively. The last two tubular elements, i.e. the inner and the median ones, are operated by a differentiated motion allowing the cutting member to continue its stroke even when the median tubular element carrying an element of the compression device stops.

2) A median portion crossed along by the central pivot, provided with mechanical means capable of imparting a slipping movement between the end portion and the central pivot during the performing of anastomosis.

3) An end portion crossed along by the central pivot, formed by a cylindrical terminal part carrying a knob able to realize, by its rotation, the slipping of the central pivot as to the whole machine. Said portion is provided with an outer scaled groove to evaluate such slipping.

The mentioned differentiated motion constitutes the main novelty of the present invention in respect of the above cited FR—A—2 490 482.

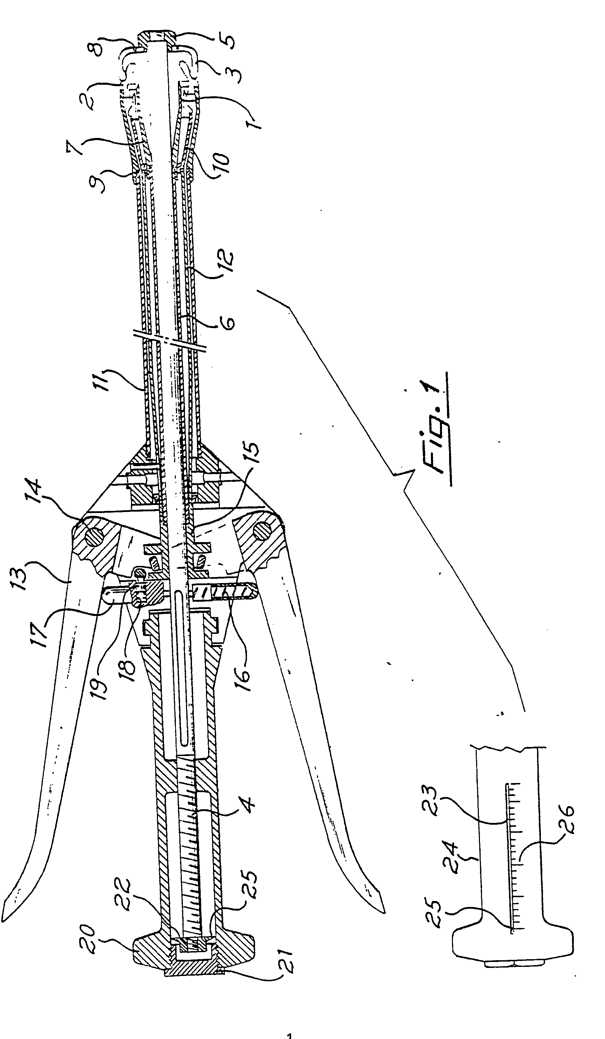

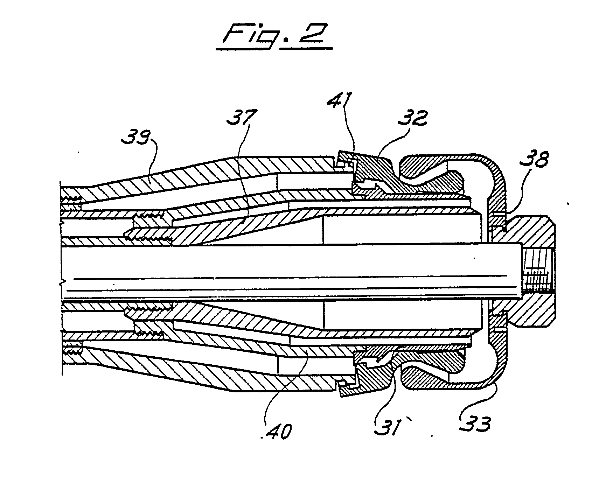

More in particular, a preferred embodiment of the invention consists in a circular mechanical anastomotic gun which will be illustrated in details in Fig. 1 and 2 of the accompanying drawings: such preferred gun however will be not considered limiting the scope of the invention.

Figure 1 represents a section view of the anastomotic gun in a rest position having inserted the compression suturing device formed by the elements 1, 2 and 3, the last two being already connected. There can be distinguished:

in the head portion, the central pivot 4 carrying the device 5 capable of stopping the element 3. The coaxial cylinder 6, inside of which the central pivot 4 runs, is the element bearing by thread the circular blade 7 which, when thrusted by the median portion device operation, comes into contact with the element 3 and cuts it along the groove 8 therein prearranged to facilitate the cut. In this way it is obtained on the element 3, a port having a diameter bigger than that of the stop element 5 so that this latter can slip through said port allowing the circular mechanical anastomotic gun be disengaged. The elements 9 and 10 are connected by thread respectively to the tubular bodies 11 and 12, which are coaxial to the central pivot 4, and carry the elements 2 and 1 respectively.

In the median portion the lever arms 13 pivoted on the pivot 14, allow operation of the body 15, being coaxial with the central pivot 4 for transmitting movement to the circular blade 7 and to the element 1. The springs 16 compressed inside the cylindrical holders 17, maintain the lever arms 13 in opening position whilst the spring 18 by means of the prong 19 operates the device safety gear.

In the end portion the central pivot 4 is partially threaded so that through rotation of the knob 20 containing inserted the locking nut 21 it may be slipped against all the device parts.

At the very end of the central pivot 4 threaded part, a cap nut 22 is made integral with it by means of thread, the cap nut 22 being so shaped that its view through the longitudinal slit 23, (detail in Fig. 1) placed on the circular member 24, consists in the index 25 evaluating on the graduated scale 26 the central pivot 4 slipping against all the other parts of the mechanical anastomotic gun.

In alternative the head portion of the mechanical circular anastomotic gun of Fig. 1, may be modified as reported in details in enlarged scale, in Figure 2: the elements 39 and 40 carrying respectively the elements 32 and 31 of the compression suturing device for effecting the anastomosis, show a different outline then corresponding elements in Fig. 1: particularly the element 39 at the end opposite to the threaded portion show an annular recess delimited outwardly by a circular ledge 41 for the engagement with a corresponding matching ledge of an element of a suitable suturing device.

The mechanical anastomotic gun showing the head portion as illustrated in Fig. 2, after the suturing device assembly has been forwarded and the cut of the tissue edges operated by means of blade 37 in connection with the groove 38 on the element 33, is separated from the assembled suturing device more easily than the gun of Fig. 1 and pulled out from the hollow organ.

In the use, the circular mechanical anastomotic gun according to the invention (reference is made in particular to the one of Fig. 1, but obviously operation is extended to the device modified as indicated in Fig. 2) is inserted through a suitable aperture into the hollow organ dissected and by a rotation of the knob 20, the elements 2 and 3 are suitably spaced each other. The purse-string suture placed on the tissue edge of the dissected organ immediately above the member 2, is tightened, then the element 3 is inserted inside the edge of the close hollow dissected organ the margins of which are stitched with another purse-string suture, and this suture tightened on the central pivot 4, immediately below the element 3. With reversed operation of the knob 20, the elements 2 and 3 are positioned one inserted into the other, then the arms of the mechanical anastomotic gun are operated to make the element 1 and the circular blade 7 shifting.

The element 1 and the circular blade 7 move together simultaneously until the element 1 reaches its final position fixing itself inside the element 2; then the circular blade 7 continues its movement and after having edged the purse-string sutures, it cuts the element 3 in connection with the groove 8.

The circular mechanical anastomotic gun is then drawn out and the suturing device remains positioned inside the hollow organ, from which it will separate only when the necrosis of the tissues occurs which stage follows the occuring of a natural suture between the tissue edges mating immediately out the area where the compression was applied.

## Claims

1. Circular mechanical anastomotic gun suitable for the insertion of an anastomotic device comprising three coaxial elements to effect the anastomosis of hollow organs, said gun comprising:

—a head portion comprising a central elongated cylindrical member (4) carrying at one end a blocking device (5) capable of supporting the outer element of the anastomotic device and, coaxial with the central elongated cylindrical member (4), successively an inner, intermediate and an outer, elongated tubular element (6, 12 and 11), which tubular elements carry at their ends facing the blocking device (5), a circular blade (7, 37) and suitably shaped supporting elements (10, 9, 40 and 39), said supporting elements being adapted to support, respectively, the inner and intermediate elements of the anastomotic device;

—a median portion comprising driving means (13, 14) for allowing movement of a body (15) coaxial with the central elongated cylindrical member (4), said body (15) transmitting its movement, thereby urging forwardly, firstly and simultaneously, the inner tubular element (6) carrying the circular blade (7, 37) and the intermediate tubular element (12) carrying the supporting element (10, 40) for the inner element of the anastomotic device, and then, after the intermediate tubular element (12) has completed its forward movement, moving further forward only the inner tubular element (6) so that the blade (7, 37) advances, cutting the outer element circumferentially outside said blocking device (5), springs (16) being provided for maintaining the driving means (13) in an open position; and

—an end portion where the central elongated cylindrical member (4) is provided with a kob (20) for shifting the elongated cylindrical member upon rotation of the knob (20) with respect to the head and median portion of the anastomotic gun.

2. Circular mechanical anastomotic gun according to claim 1, characterized in that the supporting element (39) on the outer tubular member is threaded thereto and has an annular recess delimited outwardly by a circular ledge (41), at the end opposite its threaded portion.

## Patentansprüche

1. Mechanische Pistole für die zirkuläre Anastomose, geeignet für die Einfügung einer Anastomosevorrichtung mit drei koaxialen Elementen, um die Anastomose von Hohlorganen zu bewirken, wobei die Pistole umfaßt:

—ein Kopfteil mit einem mittleren verlängerten zylindrischen Glied (4), welches an einem Ende eine Blockiervorrichtung (5) trägt, die das äußere Element der Anastomosevorrichtung und, koaxial mit dem mittleren verlängerten zylindrischen Glied (4) nacheinander ein inneres, mittleres und ein äußeres verlängertes Röhrenelement (6, 12 und 11) halten kann, wobei die Röhrenelemente an ihren der Blockiervorrichtung (5) gegenüberliegenden Enden eine kreisförmige Klinge (7, 37) und geeignet geformte Halteelemente (10, 9, 40 und 39) tragen, wobei die Halteelemente ausgelegt sind, um die inneren bzw. mittleren Elemente der Anastomosevorrichtung zu halten;

—ein mittleres Teil, welches Antriebsmittel (13, 14) umfaßt, damit eine Bewegung eines Körpers (15), koaxial mit dem mittleren verlängerten zylindrischen Glied (4) erlaubt wird, wobei der Körper (15) seine Bewegung überträgt, wodurch das innere Röhrenelement (6), welches die kriesförmige Klinge (7, 37) trägt und das mittlere Röhrenelement (12), welches das Halteelement (10, 40) für das innere Element der Anastomosevorrichtung trägt, zuerst und gleichzeitig vorwärts treibt und dann, nachdem das mittlere Röhrenelement (12) seine Vorwärtsbewegung vervollständigt hat, lediglich das innere Röhrenelement (6) weiter vorwärts bewegt, so daß die Klinge (7, 37) vorrückt, wobei das äußere Element umfangsseitig außerhalb der Blockiervorrichtung (5) geschnitten wird, Federn (16) vorgesehen sind, um die Antriebsmittel (13) in einer offenen Stellung zu halten; und

—ein Endteil, an dem das mittlere verlängerte zylindrische Glied (4) mit einem Knopf (20) zum Verschieben des verlängerten zylindrischen Glieds durch Drehung des Knopfes (20) in bezug auf das Kopf- und Mittelteil der Anastomosepistole, zur Verfügung gestellt wird.

2. Mechanische Pistole für zirkuläre Anastomose nach Anspruch 1, dadurch gekennzeichnet, daß das Halteelement (39) auf das äußere Röhrenglied angeschraubt ist und eine ringförmige Vertiefung aufweist, die nach außen durch einen kreisförmigen vorstehen Rand (41) an dem seinem Gewindeteil gegenüberliegenden Ende, begrenzt wird.

**Revendications**

1. Pistolet mécanique circulaire pour l'anastomose, convenant pour l'insertion d'un dispositif pour l'anastomose et comprenant trois éléments coaxiaux afin de réaliser l'anastomose d'organes creux ledit pistolet comportant:

—une partie de tête comportant un organe cylindrique central allongé (4) portant à une extrémité un dispositif de blocage (5) pouvant supporter l'élément extérieur du dispositif pour l'anastomose et, coaxial avec l'organe cylindrique central allongé (4), successivement un élément intérieur, un élément intermédiaire et un élément extérieur allongés et tubulaires (6, 12 et 11), lesquels éléments tubularies portent à leurs extrémités faisant face au dispositif de blocage (5) une lame circulaire (7, 37) et des éléments de support de forme adéquate (10, 9, 40 et 39), lesdits éléments de support étant adaptés pour supporter respectivement les éléments intérieur et intermédiaire du dispositif pour l'anastomose;

—une partie médiane comportant des moyens d'entraînement (13, 14) pour permettre le mouvement d'un corps (15) coaxial avec l'organe cylindrique central allongé (4), ledit corps (15) transmettant son mouvement, et de ce fait poussant vers l'avant, en premier lieu et simultanément, l'élément tubulaire intérieur (6) portant la lame circulaire (7, 37) et l'élément tubulaire intermédiaire (12) portant l'élément de support (10, 40) de l'élément intérieur du dispositif pour l'anastomose, puis, après que l'élément tubulaire intermédiaire (12) a achevé son mouvement vers l'avant, poussant davantage vers l'avant l'élément tubulaire intérieur uniquement (6) de sorte que la lame (7, 37) avance, coupant l'élément extérieur le long de la périphérie à l'extérieur dudit dispositif de blocage (5), des ressorts (16) étant prévus pour maintenir les moyens d'entraînement (13) en position ouverte; et

—une partie d'extrémité dans laquelle l'organe cylindrique central allongé (4) est muni d'un bouton (20) pour déplacer l'organe cylindrique allongé par rapport à la partie de tête et à la partie médiane du pistolet pour l'anastomose, lorsque le bouton (20) est tourné.

2. Pistolet mécanique circulaire pour l'anastomose selon la revendication 1, caractérisé en ce que l'élément de support (39) sur l'organe tubulaire extérieur est fileté à cet endroit et porte un évidement annulaire délimité vers l'extérieur par une moulure circulaire (41) à l'extrémité opposée à sa partie filetée.

_Fig. 1_

EP 0 152 382 B1

Fig. 2